# EUROPEAN PATENT SPECIFICATION

(11) **EP 4 387 538 B1**
(45) Date of publication and mention of the grant of the patent: **11.03.2026**
(21) Application number: 22859012.1
(22) Date of filing: 16.08.2022
(51) Int. Cl.: A61B 34/30, A61B 17/02, A61B 1/313, A61B 17/30, A61B 17/00

(54) **PIVOTABLE STABILIZER TOOL FOR TOTAL ENDOSCOPIC CORONARY ARTERY BYPASS**
SCHWENKBARES STABILISATORWERKZEUG FÜR TOTALEN ENDOSKOPISCHEN KORONARARTERIENBYPASS
OUTIL STABILISATEUR PIVOTANT POUR DÉRIVATION D'ARTÈRE CORONAIRE ENDOSCOPIQUE TOTALE

(30) Priority: 17.08.2021 US 202163233942 P
(43) Date of publication of application: 26.06.2024
(73) Proprietor: Terumo Cardiovascular Systems Corporation, Ann Arbor, Michigan 48103 (US)
(72) Inventor: TSUBOUCHI, Takeshi, Dexter, Michigan 48130 (US)
(74) Representative: Fish & Richardson P.C.
(86) International application number: PCT/US2022/040394
(87) International publication number: WO 2023/023007

(56) References cited:
- WO-A1-03/049659
- WO-A2-02/30352
- US-A1- 2002 045 888
- US-A1- 2003 158 463
- US-A1- 2003 158 463
- US-A1- 2017 189 003

## Description

### BACKGROUND OF THE INVENTION

The present invention relates generally to a tool for cardiac/thoracic surgery, and more specifically to a stabilization tool to restrain/stabilize a coronary area to enable anastomosis in or around a beating heart. By enabling parts of the tool to be assembled in situ, the parts of the tool are adapted to pass through small incisions/tunnels as are typically used in robotically-controlled surgeries.

The invention is especially useful for coronary bypass known as OPCABG (Off Pump Coronary Artery Bypass Grafting). OPCABG had typically been performed using a sternal procedure such as an open chest cavity, but more recently robotic surgery has become more common which can be done through small incision holes with robotic arms.

The use of minimally-invasive cardiac surgery (MICS) has become a popular method for performing cardiac repair, in order to avoid the need for open chest procedures requiring long incisions and severing of the breastbone. A working space for MICS may be created within a chest space using one or more small incisions with 1) injection of an insufflation gas, such as CO₂ gas, to expand the chest space, 2) the use of a lift winch type retractor (e.g., the Medtronic Thoratrak device which uses wire and winch mechanism to pull up chest from outside), and/or 3) in the case of mitral valve repair, a retractor blade pulled up by a shaft through chest wall (e.g., the Atricure atrial lift).

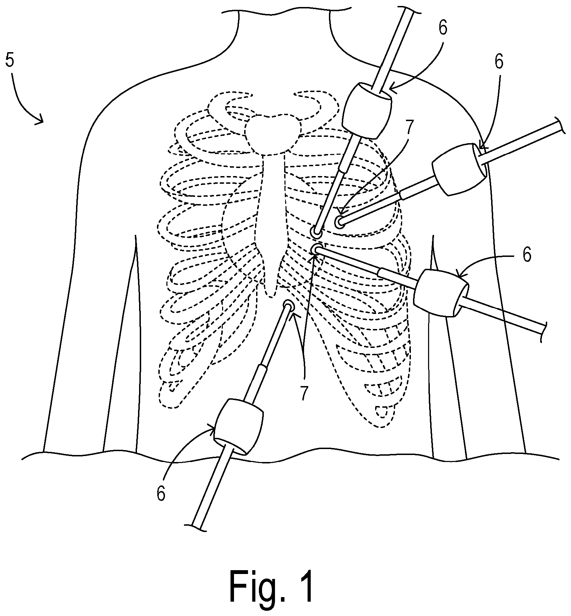

One particular type of MICS known as total endoscopic coronary artery bypass (TECAB) may be performed through four to five small slits (i.e., ports) for entry by several robotically controlled instruments and other support devices into the coronary region. Figure 1 shows a patient 5 with multiple instruments 6 inserted via different respective incised holes (i.e., tunnels) 7 to access an internal working space. The da Vinci Surgical System is one example of a robotic system used for this type of closed-chest operation.

A robotic system includes robotic arms that are manipulated by the surgeon which may include a camera, cutting tools, grasping tools, and suturing tools, for example. Preferably, surgery may be performed as the heart continues to beat, so that no heart-lung bypass machine is required. With the heart beating, it becomes desirable to restrain or stabilize the area around the heart. In order to obtain a highly effective stabilization, stabilizer members having suction for adhering to the surface (e.g., cardiac tissue surface) to be stabilized may be preferred. A support member (e.g., robotic arm) or other stationary fixture holds the stabilizer members in a desired location once the suction has been activated. A cardiac stabilizer tool for stabilizing an area for anastomosis needs to go through a small hole (e.g., about 12mm) in order to be deployed inside of the body. It has been very difficult to insert stabilization tools of sufficient dimensions and with suction capability into the small holes used in TECAB and other procedures. Examples of prior art procedures and tools are shown in US patent 6,936,001 and US patent 8,870,900.

US 2017/0189003 describes a minimally invasive heart stabilizer includes a guide tube, a revolute joint, a stabilizing end-effector, and a linkage. The guide tube is sized to allow insertion through an endoscopic cannula. The revolute joint is coupled to a distal end of the guide tube. The stabilizing end-effector is coupled to the revolute joint. The linkage has a distal end and a proximal end. The distal end is pivotally connected to the end-effector. The stabilizing end-effector includes two tissue engaging members in parallel alignment with one another. The two tissue engaging members are joined via an arrangement of links.

US 2003/0158463 describes a tissue stabilizer for endoscopically stabilizing a target tissue within a patient's body.

WO 03/049659 describes a heart stabilizer that includes a wrist which couples an end effector to a first linkage. The end effector and wrist may be inserted through an incision in the chest of a patient to assist in performing a minimally invasive coronary procedure.

### SUMMARY OF THE INVENTION

The invention realizes the desired functions easily by separating elements of the tool for insertion and providing a holding mechanism adapted for robotic surgery and port surgery (e.g., small hole surgery with a manually inserted long shaft tool) to assemble and then constrain the parts. Thus, the stabilizing tool is insertable in a small number of pieces (e.g., two pieces) through a respective side hole or holes (e.g., from a port in a lateral side of the patient's body).

More specifically, a pair of stabilizer members may have the form of suction paddles or feet which may be assembled side-by-side at the end of a stabilizer (e.g., robotic) arm. The suction paddles may be inserted serially through the small hole (while connected to the end of a respective suction tube) so that they can fit through the hole one at a time. Once inside a working space within the body of the patient, there is sufficient room in the chest cavity for robotically manipulating the members and grasping them into a configuration that performs the stabilization function.

According to the invention, a stabilizer is provided for minimally invasive cardiac surgery. A stabilizer member has a suction pod at a distal end and a mounting section at a proximal end, wherein the mounting section includes a fixed tab and a rotatable lever arm with a movable tab on a first end of the rotatable lever arm. The tabs are configured to extend through an opening in a respective jaw of a robotic arm. A lever operator is coupled to the rotatable lever arm and is configured to pivot the rotatable lever arm between an expanded state of the tabs and a contracted state of the tabs, wherein the contracted state allows the tabs to pass through the opening in the respective jaw, and wherein the expanded state of the tabs locks the stabilizer member to the respective jaw. In some embodiments, the lever operator may be a drawstring anchored to a second end of the lever arm, wherein displacing the drawstring in a proximal direction moves the movable tab of the lever arm away from the fixed tab to the expanded state which captures the respective jaw.

### BRIEF DESCRIPTION OF THE DRAWINGS

Figure 1 is a schematic diagram showing deployment of instruments in multi-port cardiac surgery.
Figure 2 is a top view of a stabilizer tool as assembled after insertion into an internal working space.
Figure 3 is a front view of the stabilizer tools of Figure 2.
Figure 4 shows the stabilizer tool of Figure 3 with stabilizer members separated from the jaws of a robotic arm.
Figure 5 is a bottom, perspective view of the arrangement shown in Figure 3.
Figure 6 is a horizontal cross-sectional view of the stabilizer members captured on the jaws.
Figure 7 is an exploded view of a stabilizer member.
Figure 8 is a cross-sectional view of the stabilizer member of Figure 7.
Figure 9 is a cross-sectional view of the stabilizer member in an expanded state.

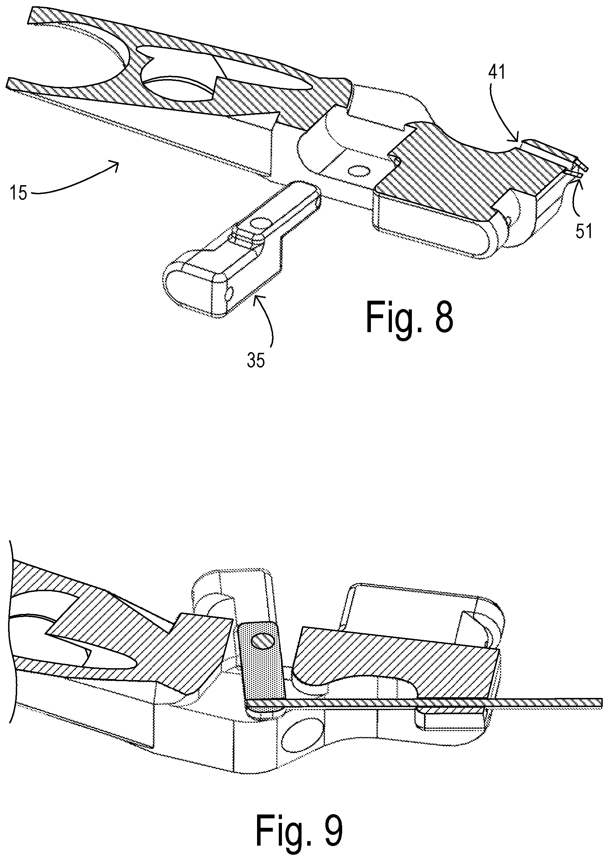

Figure 9 is side view showing a portion of the stabilizer member and a location for cutting a drawstring to allow the stabilizer member to move from an expanded state to a contracted state.

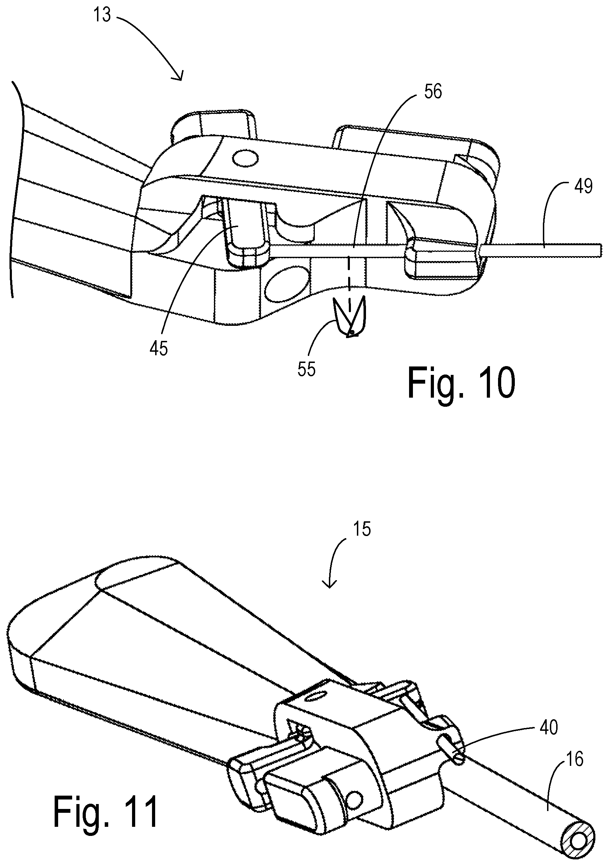

Figure 11 shows the stabilizer member in a contracted state for passing through a hole in the jaw.

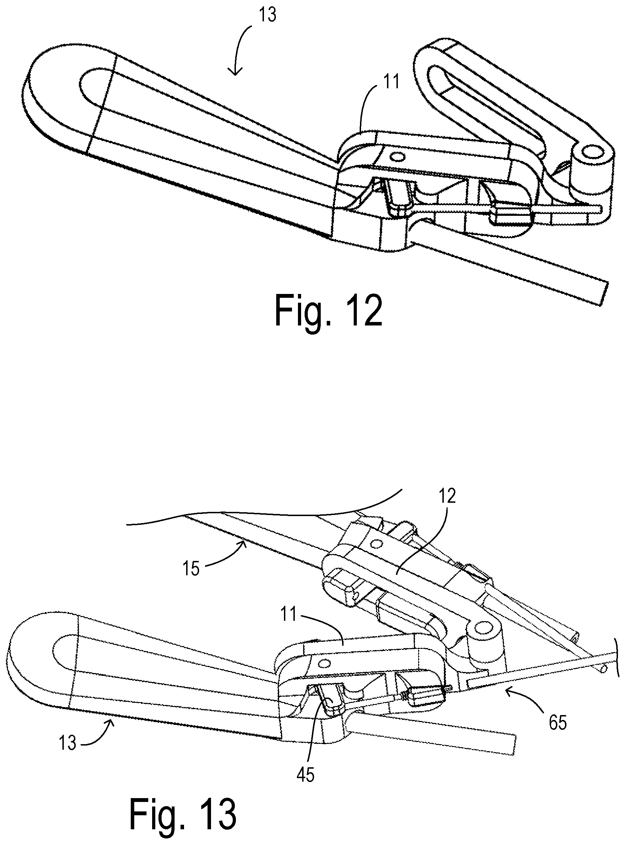

Figure 12 shows the stabilizer member in an expanded state after insertion into the jaw to grasp the jaw.
Figure 13 is a perspective view showing another embodiment of a stabilizer assembly.

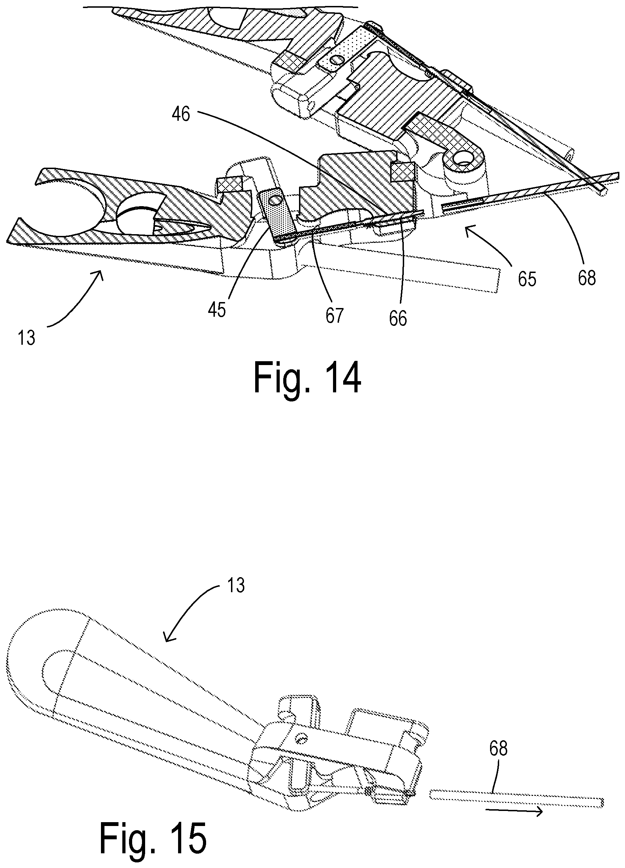

Figure 14 is a cross-sectional view of the stabilizer member in the position shown in Figure 13.
Figure 15 shows the screw adjustment of Figures 13 and 14 in greater detail.

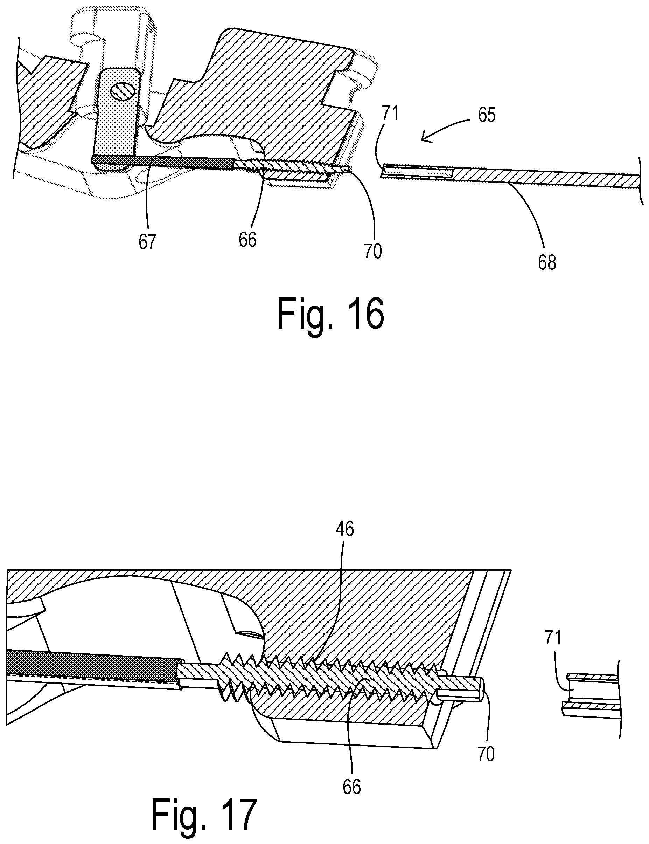

Figure 16 and 17 are cross-sectional views showing the screw adjustment of Figures 13 and 14 in greater detail.

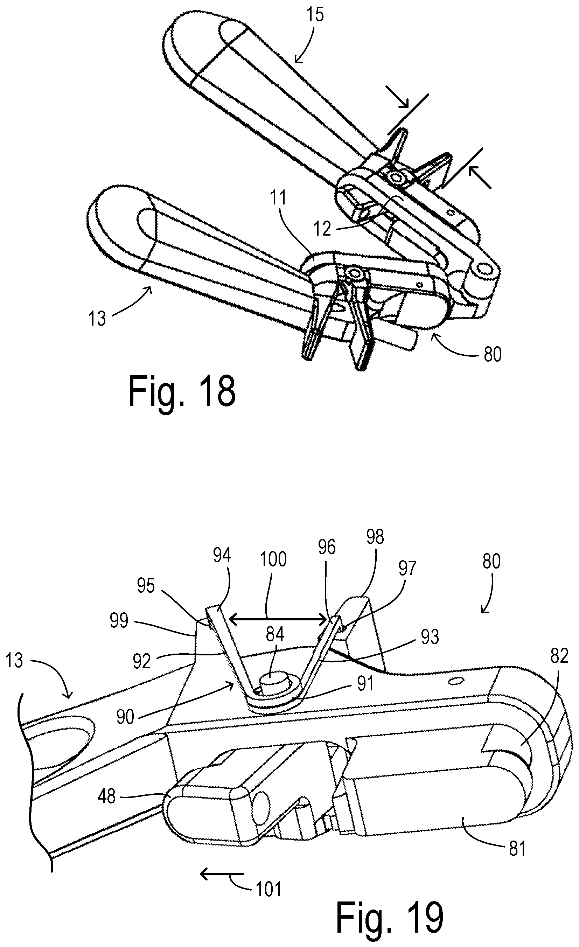

Figure 18 is a perspective view of a third embodiment of a stabilizer tool which is spring loaded.
Figure 19 is a bottom, perspective view of a stabilizer member showing a spring for biasing the stabilizer member into an expanded state.

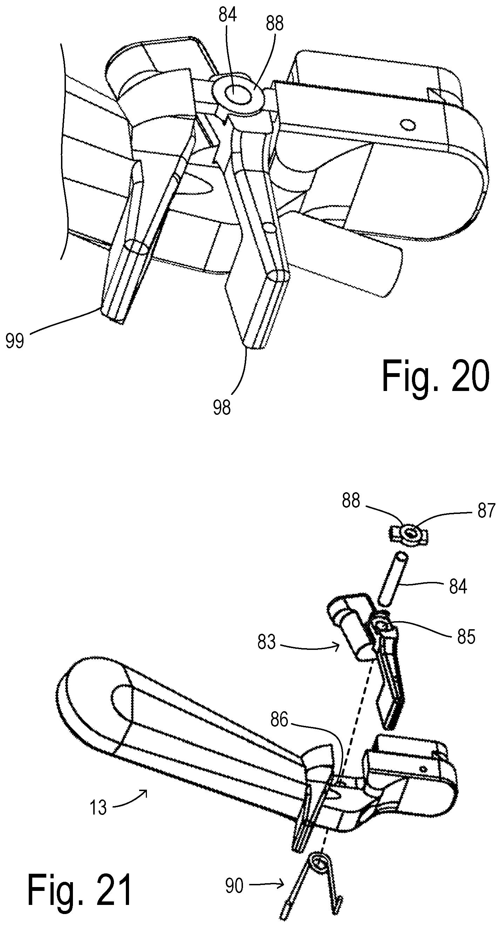

Figure 20 is a side view of the stabilizer member showing grasping tabs in greater detail.
Figure 21 is an exploded view of the stabilizer tool of Figure 18.

### DETAILED DESCRIPTION OF THE PREFERRED EMBODIMENTS

A typical robotic arm (e.g., a robotic tool or other MICS grasping instrument) may be fitted with a pair of scissor-like jaws which can be remotely opened and closed (i.e., pivoted). The jaws may be interchangeable and can have various shapes (such as a flat, paddle shape) depending on the functions to be performed. The jaws may have aligned central openings so that each forms a loop, which assists in grasping and holding onto tissue surfaces.

The left and right stabilizer members of the invention are adapted to be held separately by a respective jaw, so that their suction paddles can assume a range of separation distances by pivoting the jaws in the usual manner. Each stabilizer member includes an adjustable mechanism for mounting to a respective jaw of a robotic arm in a robust manner so that beating heart tissue can be held relatively motionless by the suction paddles of the stabilizer members.

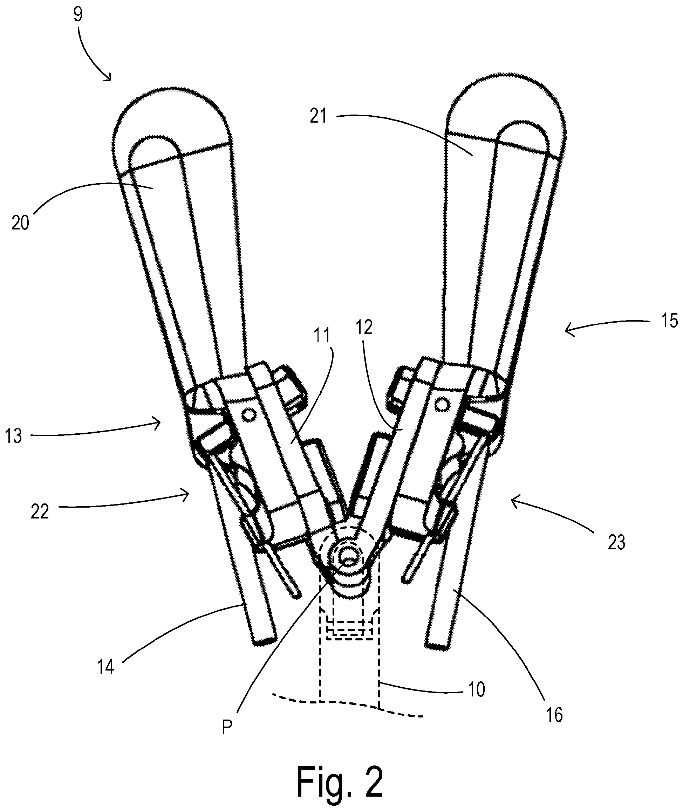

A first embodiment of a stabilizer assembly and robotic arm is shown in Figures 2-12. A robotic arm 10 has a pair of distal jaws 11 and 12 held by a remote-controlled pivot mechanism P which enables jaws 11 and 12 to be rotated together and apart. The distal end of arm 10 which supports jaws 11 and 12 can also be articulated at other degrees of freedom to interact with various tissues and other devices after being inserted into a working space within a human body via a small diameter hole (e.g., 12mm). A stabilizer assembly 9 has a first stabilizer member 13 connected to a distal end of a suction tube 14 and a second stabilizer arm 15 connected to a distal end of a suction tube 16. Suction tubes 14 and 16 extend out through the incised holes (i.e., only a distal end section is shows for each tube). Each stabilizer member has distal feet or pods with suction openings on a lower side to engage the tissue to be stabilized. Internal channels interconnect the suction openings to tubes 14 and 16 which are coupled to a suction source outside the body. A proximal end of robotic arm 10 may be attached to a patient table or other fixed structure by mounting structure (e.g., an articulating and/or telescoping stabilizer arm that grasps the shaft of arm 10 and allows it to be moved and secured at an optimal position).

Figure 2 shows the two stabilizer members 13 and 15 mounted on respective jaws in the relationship they would have inside the working space. Figure 11 shows stabilizer member 15 separate from robotic arm 10 as it would be inserted through a port or ports into the working space. Arm 10 may typically be inserted through a different port than the stabilizer members. Attachments of stabilizer members 13 and 15 to robotic arm 10 may typically be accomplished using additional robotic arms inserted through the same or different ports.

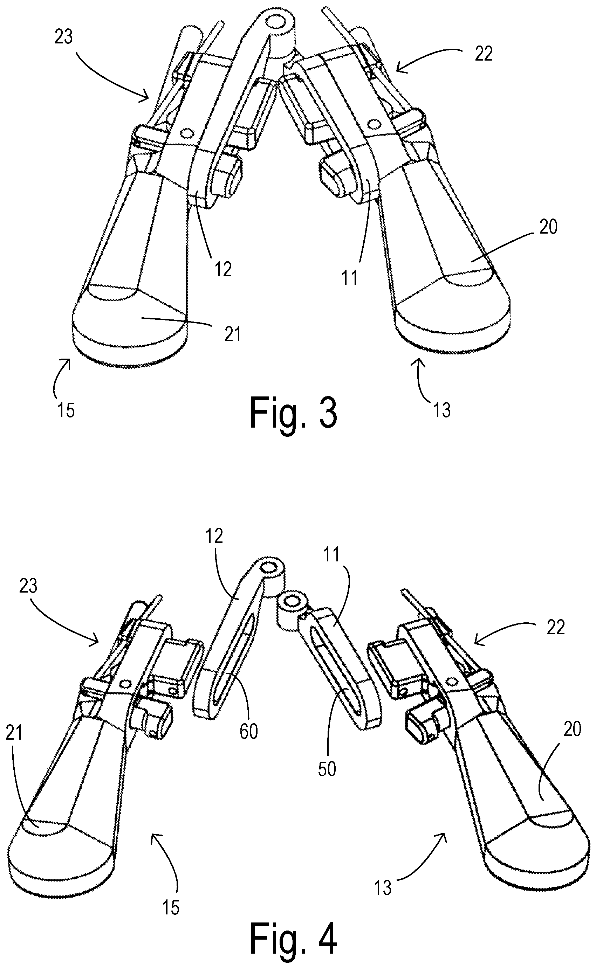

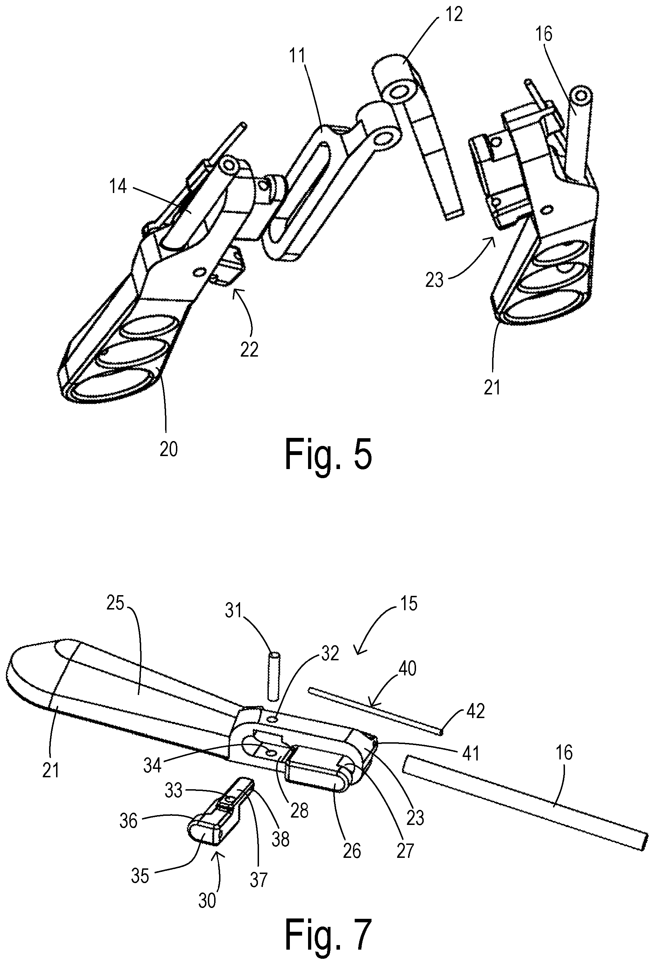

Figures 3-10 show the first embodiment of stabilizer members 13 and 15 and jaws 11 and 12 in greater detail. Stabilizer members 13 and 15 have suction pods or feet 20 and 21 at their distal ends and adjustable mounting sections 22 and 23 at their proximal ends. Figure 7 shows an exploded view of stabilizer member 15 (member 13 has the same components and is symmetrical, e.g., a mirror image). A main body 25 may be formed as a unitary body (e.g., molded plastic body or cast metal body) which has suction foot 21 at the distal end and mounting section 23 at the proximal end. Mounting section 23 has a fixed tab 26 with an end slot 27 on its proximal side. An aperture 28 extends through body 25 at the distal side of tab 26. A lever arm 30 is mounted in aperture 28 for rotation around a pivot pin 31 which passes through a hole 32 in body 25, a hole 33 in lever arm 30, and a hole 34 in body 25. Pin 31 is held by interference fit in holes 32 and 34, while hole 33 has clearance to allow pivoting of lever arm 30 around the axis of pin 31.

Lever arm 30 has a first end 35 extending out from aperture 28 beside tab 26 and a second end 37 extending out from aperture 28 on the opposite side. Second end 37 has a mounting hole 38 or other attachment point for receiving a distal end of a drawstring 40. Drawstring 40 may be a ligature (e.g., a long, bendable tether) which may be comprised of a thread, string, fiber cord, plastic line, or metal wire, for example. Drawstring 40 passes through a passage 41 in the proximal end of body 25 and has a proximal end 42 (e.g., extending outside the body through the incised hole) for grasping and pulling in order to rotate lever arm 30. First end 35 has a flange 36 extending transversely. When first end 35 is rotated to a position closest to tab 26, they together fit through a center opening 50 in jaw 12 (see Figure 4). Likewise, jaw 11 has an opening 60.

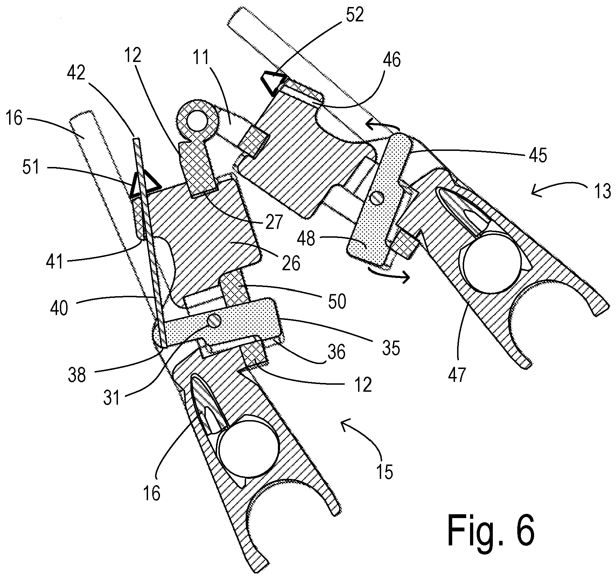

Figure 6 is a top cross-sectional view in a condition after which tab 26 and flange 36 have been inserted through opening 50 in jaw 12. Drawstring 40 has been pulled so that first end 35 has moved away (i.e., spread apart) from tab 26. Jaw 12 is locked into end slot 27 and beneath flange 36 so that member 15 is firmly held on jaw 12. Similarly, stabilizer member 13 has a lever arm 45. A drawstring (not shown in Figure 6 but shown as drawstring 49 in Figure 10) for lever arm 45 passes through a passage 46 in a main body 47 of member 13. Pulling on one side of lever arm 45 with the drawstring rotates it in the direction shown by arrows so that a flange 48 captures jaw 11.

Once a drawstring is pulled in order to capture a stabilizer member onto a jaw, it can be secured by a tension lock or tie-down so that tension remains on the drawstring without continued pulling. For example, a tension lock 51 is disposed on a proximal end of member 15 (Figure 8) and a tension lock 52 is disposed on a proximal end of member 13 (Figure 6), each having one or more flexible walls (e.g., a funnel shape or two or more opposing flaps) sloping in the proximal direction and defining a central opening with a diameter slightly smaller than a diameter of the drawstring. Thus, the drawstring can be easily pulled in a proximal direction but resists movement in the distal direction through locks 51 and 52. After pulling on a drawstring to capture a stabilizer member onto a jaw, the proximal end of the drawstring can be released, and the stabilizer jaw remains locked to the jaw. In other words, tension locks 51 or 52 are configured to allow one-way movement of drawstring 40 through the tension lock so that when lever arm 45 is pulled from the contracted state to the expanded state then the expanded state is preserved without further pulling on drawstring 40.

As shown in Figure 10, in order to release a stabilizer member from a jaw after completing a relevant portion of a surgical procedure, a cutting device 55 can be used to sever drawstring 49 at a location 56 between lever arm 45 and lock 52. Once the tension is released, a robotic tool can counter-rotate lever arm 45 (or a bias spring can be incorporated with lever arm 45 to automatically counter rotate it) and the stabilizer member can be removed from the body through the corresponding port.

Figure 11 shows stabilizer member 15 in an unconnected state which facilitates insertion and removal through the surgical port. After insertion, each stabilizer member is coupled to a respective jaw one at a time. Figure 12 shows stabilizer member 13 initially coupled onto jaw 11.

Figures 13-17 show an alternative embodiment wherein a drawstring for pivoting a lever arm utilizes a threaded mechanism. Other aspects of stabilizer members 13 and 15 may be identical to the previous embodiment, and their description will not be repeated. A compound drawstring 65 includes a threaded bar 66 affixed to a ligature 67 (e.g., a cord) and to a torque wire 68 (see Figures 14 and 16). Ligature 67 is captured by lever arm 45 in stabilizer member 13. Passage 46 in the main body of stabilizer member 13 is threaded so that rotation of threaded bar 66 causes it to advance within passage 46 and apply tension to ligature 67 that pulls lever arm 45 from the unlocked position (i.e., contracted state) into the locked position (i.e., expanded state). Ligature 67 may be composed of any of the materials mentioned above (e.g., yarn, plastic, metal) for the drawstring. Bar 66 and wire 68 may be a biocompatible metal or molded plastic material.

As shown in Figures 16 and 17, at a proximal side of threaded bar 66 there is a keyed end 70 having a male, hexagonal projection for driving the rotation. Torque wire 68 has a female hexagonal socket 71 that captures keyed end 70. By grasping and rotating torque wire 68 (e.g., using a robotic arm having a gripper or forceps), bar 66 may be rotated counterclockwise to pull lever arm 45 to the locked position. Optionally, a holding force of socket 71 on keyed end 70 may be configured to allow torque wire 68 to be detached by pulling it off of bar 66 after the locking is performed (Figure 15). This can allow the space occupied by the stabilizer apparatus to be reduced during the remainder of the surgery.

Ligature 67 may be affixed to bar 66 by adhesive bonding, for example. In order to remove the stabilizer members after a cardiac repair, ligature 67 can be severed so that the stabilizer members can be removed from the jaws of the robotic arms. In addition to mechanical cutting as described above, ligature 67 can be severed using electrocautery (which may be especially convenient since electrocautery tools are typically used as part of the cardiac surgery). Ligature 67 may be a conductive material such as copper or a mixture of carbon and/or metal with a plastic yarn, for example. Ligature 67 is made sufficiently resistive such that a current from the electrocautery tool causes heating which melts/severs ligature 67.

Figures 18-21 show a third embodiment which utilizes a spring to bias a mounted section into an expanded state. Specifically, a mounting section 80 at a proximal end of stabilizer member 13 has a fixed tab 81 defining an end slot 82 (for receiving a respective side of the jaw). A lever arm 83 is received in a slot in mounting section 80 so that lever arm 83 is rotatable around a pivot pin 84. Pin 84 passes through a pivot hole 85 in lever arm 83 which has a diameter which permits movement of lever arm 83. A lower end of pin 84 is affixed in a mounting hole 86, and an upper end of pin 84 is affixed in a mounting hole 87 of a fastening block 88 which closes an upper side of the lever arm slot. Thus, lever arm 83 is movable between a contracted state and an expanded state so that flange 48 can controllably lock to a jaw as previously described.

In order to bias lever arm 83 to an expanded state, a bias spring 90 is provided. Bias spring 90 has a center hub 91 and spring arms 92 and 93. Center hub 91 is shaped as a coil which receives an end of pivot pin 84 that extends beyond a lower surface of mounting section 80. Spring arm 92 has an end 94 which is affixed in a stationary hole 95 in a lower surface of a fixed extension 99. Spring arm 93 has an end 96 affixed in a moving hole 97 in a moving extension 98 which is part of lever arm 83. Spring 90 is configured such that hub 91 is compressed due to the attachments of spring arms 92 and 93. The compression creates an expansion force 100 between extensions 98 and 99 which biases lever arm 83 in the expanded state with flange 48 moved in a direction 101 to a locking position.

As shown in Figure 18, an external compression force can be applied to the extensions in order to overcome expansion force 100. For example, the extensions can be squeezed together by a forceps or other tool inserted in the working space. The external compression force causes lever arm 83 to move into the contracted state. The same forceps can be used to manipulate a stabilizer member in order to pass the tabs through the center hole in a respective jaw (either for insertion or removal). When inserting the tabs onto a jaw, then once the tabs are in place then the forceps can be opened and the spring action will expand the tabs and lock the stabilizer member onto the jaw.

## Claims

1. A stabilizer for minimally invasive cardiac surgery comprising:
a stabilizer member (13, 15) having a suction pod (20, 21) at a distal end and a mounting section (22, 23) at a proximal end, wherein the mounting section includes a fixed tab (26) and a movable tab (36) on a first end of a rotatable lever arm (30, 45), wherein the tabs are configured to extend through an opening (50, 60) in a respective jaw of a robotic arm; and
a lever operator (40, 49) coupled to the rotatable lever arm configured to pivot the rotatable lever arm between an expanded state of the tabs and a contracted state of the tabs,
**characterised in that**
the contracted state allows the tabs to pass through the opening in the respective jaw, and wherein the expanded state of the tabs locks the stabilizer member to the respective jaw.

2. The stabilizer of claim 1 wherein the lever operator is comprised of a drawstring anchored to a second end of the lever arm, wherein displacing the drawstring in a proximal direction moves the movable tab of the lever arm away from the fixed tab to the expanded state which captures the respective jaw.

3. The stabilizer of claim 2 wherein the drawstring is comprised of a ligature passing through a fixed portion of the stabilizer member.

4. The stabilizer of claim 2 wherein the drawstring is comprised of:
a ligature (67) captured by the lever arm;
a threaded bar (66) connected to the ligature and passing through a threaded passage in a fixed portion of the stabilizer member; and
a torque wire (68) configured to hold an end of the threaded bar such that rotation of the torque wire pulls the ligature to move the lever arm.

5. The stabilizer of claim 2 further comprising:
a tension lock (51, 52) receiving the drawstring configured to allow one-way movement of the drawstring through the tension lock so that when the lever arm is pulled from the contracted state to the expanded state then the expanded state is preserved without further pulling on the drawstring.

6. The stabilizer of claim 2 wherein the drawstring is configured to be severed to release the lever arm for returning to the contracted state.

7. The stabilizer of claim 1 wherein the lever operator is comprised of a bias spring (90) configured to urge the lever arm into the expanded state.

8. The stabilizer of claim 7 wherein the bias spring is comprised of a coil at a center hub (91) and a pair of spring arms (92, 93), wherein one spring arm is affixed to a second end of the lever arm and the other spring arm is affixed to a fixed portion of the stabilizer member.

9. The stabilizer of claim 8 wherein the second end of the lever arm forms a moving extension, wherein the stabilizer member defines a fixed extension juxtaposed to the moving extension, wherein the extensions are configured to be squeezed together to compress the bias spring and move the lever arm into the contracted state.

10. The stabilizer of claim 1 further comprising a second stabilizer member and a second drawstring configured to capture a second jaw of the robotic arm so that the stabilizer members are arranged side by side and have an adjustable distance between them.

## Patentansprüche

1. Stabilisator für minimal-invasive Herzchirurgie, umfassend:
ein Stabilisatorelement (13, 15) mit einem Saugkopf (20, 21) an einem distalen Ende und einem Montageabschnitt (22, 23) an einem proximalen Ende, wobei der Montageabschnitt eine feststehende Lasche (26) und eine bewegliche Lasche (36) an einem ersten Ende eines drehbaren Hebelarms (30, 45) aufweist, wobei die Laschen so ausgelegt sind, dass sie sich durch eine Öffnung (50, 60) in einer entsprechenden Backe eines Roboterarms hindurch erstrecken, und
ein an den drehbaren Hebelarm gekoppeltes Hebelbedienelement (40, 49), das so ausgelegt ist, dass es den drehbaren Hebelarm zwischen einem expandierten Zustand der Laschen und einem zusammengezogenen Zustand der Laschen dreht,
**dadurch gekennzeichnet, dass**
der zusammengezogene Zustand gestattet, dass die Laschen durch die Öffnung in der jeweiligen Backe gehen, und wobei das Stabilisatorelement durch den expandierten Zustand der Laschen an der jeweiligen Backe verriegelt wird.

2. Stabilisator nach Anspruch 1, wobei das Hebelbedienelement aus einem Zugband besteht, das an einem zweiten Ende des Hebelarms verankert ist, wobei die bewegliche Lasche des Hebelarms durch das Verschieben des Zugbands in einer proximalen Richtung von der feststehenden Lasche weg in den expandierten Zustand bewegt wird, wodurch die jeweilige Backe erfasst wird.

3. Stabilisator nach Anspruch 2, wobei das Zugband aus einer Ligatur besteht, die durch einen feststehenden Abschnitt des Stabilisatorelements geht.

4. Stabilisator nach Anspruch 2, wobei das Zugband aus Folgendem besteht:
einer von dem Hebelarm erfassten Ligatur (67),
einer mit der Ligatur verbundenen Gewindestange (66), die durch einen Gewindedurchgang in einem feststehenden Abschnitt des Stabilisatorelements geht, und
einem Drehmomentdraht (68), der dazu ausgelegt ist, ein Ende der Gewindestange so zu halten, dass durch die Drehung des Drehmomentdrahts an der Ligatur gezogen wird, um den Hebelarm zu bewegen.

5. Stabilisator nach Anspruch 2, ferner umfassend:
eine das Zugband aufnehmende Spannverriegelung (51, 52), die so ausgelegt ist, dass sie eine gerichtete Bewegung des Zugbands durch die Spannverriegelung gestattet, so dass, wenn der Hebelarm aus dem zusammengezogenen Zustand in den expandierten Zustand gezogen wird, der expandierte Zustand dann erhalten bleibt, ohne dass weiter an dem Zugband gezogen wird.

6. Stabilisator nach Anspruch 2, wobei das Zugband so ausgelegt ist, dass es abgetrennt wird, um den Hebelarm für die Rückkehr in den zusammengezogenen Zustand freizugeben.

7. Stabilisator nach Anspruch 1, wobei das Hebelbedienelement aus einer Vorspannfeder (90) besteht, die dazu ausgelegt ist, den Hebelarm in den expandierten Zustand zu drängen.

8. Stabilisator nach Anspruch 7, wobei die Vorspannfeder aus einer Spule an einer Mittelnabe (91) und einem Paar Federarme (92, 93) besteht, wobei ein Federarm an einem zweiten Ende des Hebelarms befestigt ist und der andere Federarm an einem feststehenden Abschnitt des Stabilisatorelements befestigt ist.

9. Stabilisator nach Anspruch 8, wobei das zweite Ende des Hebelarms eine bewegliche Verlängerung bildet, wobei das Stabilisatorelement eine feststehende Verlängerung definiert, die der beweglichen Verlängerung gegenüberliegt, wobei
die Verlängerungen zum Zusammendrücken ausgelegt sind, um die Vorspannfeder zu komprimieren und den Hebelarm in den zusammengezogenen Zustand zu bewegen.

10. Stabilisator nach Anspruch 1, ferner umfassend ein zweites Stabilisatorelement und ein zweites Zugband, das zum Erfassen einer zweiten Backe des Roboterarms ausgelegt ist, so dass die Stabilisatorelemente nebeneinander angeordnet sind und einen verstellbaren Abstand zwischen sich haben.

## Revendications

1. Stabilisateur pour chirurgie cardiaque peu invasive comprenant :
un élément stabilisateur (13, 15) comportant une capsule d'aspiration (20, 21) à une extrémité distale et une section de montage (22, 23) à une extrémité proximale, la section de montage comprenant une patte fixe (26) et une patte mobile (36) sur une première extrémité d'un bras de levier rotatif (30, 45), les pattes étant configurées pour s'étendre à travers une ouverture (50, 60) dans une mâchoire respective d'un bras robotique ; et
un actionneur de levier (40, 49) couplé au bras de levier rotatif configuré pour faire pivoter le bras de levier rotatif entre un état déployé des languettes et un état contracté des languettes,
**caractérisé en ce que**
l'état contracté permet aux languettes de passer à travers l'ouverture dans la mâchoire respective, et l'état déployé des languettes verrouille l'élément stabilisateur sur la mâchoire respective.

2. Stabilisateur selon la revendication 1, l'opérateur de levier étant constitué d'un cordon de serrage ancré à une seconde extrémité du bras de levier, le déplacement du cordon de serrage dans une direction proximale déplaçant la patte mobile du bras de levier en l'éloignant de la patte fixe vers l'état déployé qui capture la mâchoire respective.

3. Stabilisateur selon la revendication 2, le cordon de serrage étant constitué d'une ligature passant à travers une partie fixe de l'élément stabilisateur.

4. Stabilisateur selon la revendication 2, le cordon de serrage étant constitué de :
une ligature (67) capturée par le bras de levier ;
une barre filetée (66) reliée à la ligature et passant à travers un passage fileté dans une partie fixe de l'élément stabilisateur ; et
un fil de torsion (68) configuré pour maintenir une extrémité de la barre filetée de sorte que la rotation du fil de torsion tire la ligature pour déplacer le bras de levier.

5. Stabilisateur selon la revendication 2, comprenant en outre :
un verrou de tension (51, 52) recevant le cordon de serrage configuré pour permettre un mouvement unidirectionnel du cordon de serrage à travers le verrou de tension de sorte que lorsque le bras de levier est tiré de l'état contracté à l'état déployé, l'état déployé est alors conservé sans tirer davantage sur le cordon de serrage.

6. Stabilisateur selon la revendication 2, le cordon de serrage étant configuré pour être sectionné pour libérer le bras de levier pour revenir à l'état contracté.

7. Stabilisateur selon la revendication 1, l'actionneur de levier étant constitué d'un ressort de sollicitation (90) configuré pour pousser le bras de levier dans l'état déployé.

8. Stabilisateur selon la revendication 7, le ressort de sollicitation étant constitué d'une bobine au niveau d'un moyeu central (91) et d'une paire de bras de ressort (92, 93), un bras de ressort étant fixé à une seconde extrémité du bras de levier et l'autre bras de ressort étant fixé à une partie fixe de l'élément de stabilisateur.

9. Stabilisateur selon la revendication 8, la seconde extrémité du bras de levier formant une extension mobile, l'élément stabilisateur définissant une extension fixe juxtaposée à l'extension mobile,
les extensions étant configurées pour être comprimées l'une contre l'autre afin de comprimer le ressort de sollicitation et de déplacer le bras de levier dans l'état contracté.

10. Stabilisateur selon la revendication 1, comprenant en outre un second élément stabilisateur et un second cordon de serrage configurés pour capturer une seconde mâchoire du bras robotique de sorte que les éléments stabilisateurs sont agencés côte à côte et ont une distance réglable entre eux.
